# EUROPEAN PATENT SPECIFICATION

(11) **EP 2 734 550 B1**
(45) Date of publication and mention of the grant of the patent: **15.03.2017**
(21) Application number: 12751370.3
(22) Date of filing: 12.07.2012
(51) Int. Cl.: C07K 16/26, A61P 5/26, A61K 39/40, A61K 35/74

(54) **A TOPICAL COMPOSITION FOR THE TREATMENT OF ALOPECIA**
TOPISCHE ZUSAMMENSETZUNG ZUR BEHANDLUNG VON ALOPEZIE
COMPOSITION TOPIQUE POUR TRAITER L'ALOPÉCIE

(30) Priority: 18.07.2011 IT FI20110140
(43) Date of publication of application: 28.05.2014
(73) Proprietor: IN Fieri S.r.l., 50129 Firenze (IT)
(72) Inventor: VOLPATO, Ivo, I-06070 S. Mariano (Perugia) (IT); BIZZINI, Bernard, F-11000 Carcassonne (FR); GIOVANNINI, Enore, I-35010 Vigonza (Padova) (IT)
(74) Representative: Brazzini, Silvia
(86) International application number: PCT/IB2012/053573
(87) International publication number: WO 2013/011431

(56) References cited:
- EP-A1- 0 433 744
- EP-A1- 0 640 690
- EP-A1- 1 234 584
- EP-A1- 1 243 256
- EP-A1- 1 344 529
- WO-A2-2008/009960
- CH-A5- 640 141
- FR-A1- 2 700 470

## Description

### Field of the invention

The present invention relates to the field of the products for the treatment of alopecia; more specifically, subject of the invention is a composition for topical use in this kind of treatment.

### State of the art

It is nowadays well known [Ganong, Trattato di Fisiologia Medica, 1973; R. Selleri et al., Chimica e Tecnologia dei Prodotti Cosmetici, 1977; P.S. Bekhor, Austr. Fam. Phys., 1986, 15(7), 868] that the hair loss and baldness are caused by alterations in the steroid mechanisms, and in the glucidic metabolic and autocrine-paracrine mechanisms, which regulate the life of hair, and that various factors may contribute to this alteration; amongst these factors the most common are: genetic predisposition, hormonal factors, dysmetabolic factors, secondary pathologies, neoplasiae, autoimmune diseases, physical-chemical stress, etc.

More particularly, for the most common classes of alopecia it was for instance shown that: the androgenetic alopecia (common baldness) is due to a genetic message in need of androgenic hormones, in the absence of which this type of baldness does not occur; the "alopecia areata", characterized by the presence of patches subject to possible spontaneous remission, is considered as an autoimmune disease having auto-antibodies pathogenesis, mainly affecting the hair follicles; the cicatricial alopecia is the fibrotic scarring result of a diverse group of pathological processes involving the destruction of the hair follicle. Furthermore, the total or partial absence of hair may also occur as an isolated genetic factor or in association with anomalies of other organs or with abnormalities of the hair strand. To these abnormalities, in particular as regards the consistency of the scalp, the atrophy of hair follicles, of the dermis and of the subcutaneous tissue is associated, due to aging or other causes of different nature, that leads to a progressive slowing down of the renewal processes up to a thinning of hair in all areas of the skin.

The reduced functionality of the hair follicles leads, as a consequence, to a reduced elimination of the sebum produced by the sebaceous glands with its consequent accumulation in the bulb orifice. The sebum, whose physiological function is to form a hydrolipidic protective filter for the skin surface and to lubricate and seal the outer surface of the hair, tends however to harden, also due to in situ accumulation of extraneous powdery material and of keratin residues; this prevents a correct "oxygenation" and the consequent functional replacement at the site of reproduction of the hair, thus triggering of a cascade reaction of hair loss. Therefore, among the causes of hair loss, there are also the alterations in the secretion of sebum, which is controlled by the steroid hormones, either circulating or produced by the pilosebaceous follicle starting from precursors of gonadal and adrenal origin, among which the most active appears to be the androstandiol.

It is therefore a principle now acquired that the simultaneous and synergistic adjustment of all the alterations of the main mechanisms involved is necessary to counter hair loss and facilitate the regrowth process, that means to act mainly through: elimination of testosterone and active metabolites in excess in the hair follicle; reactivation of cells and tissues functionality, always in the follicle; facilitating the elimination of sebum thus preventing its accumulation and hardening; reactivation of keratinocytes function with normalization of endogenous biosynthesis of HrGF (Hair Growth Factor; growth factor of human hair).

From the analysis of the state of the art on therapeutics used so far in the treatment of alopecia of different aetiology, it clearly emerges that until today no formulations were developed able to provide synergistically all these properties and, consequently, to achieve satisfactory clinical results.

Among the classic and traditional therapies the use of placental extracts, rubefacients and certain plant extracts, such as nettle, is disclosed; whereas among the so-called current and emerging therapies, are estrogens and progestins, drugs such as cyproterone, spiranolactone, cimetidine, flutamide, ketoconazole, minoxidil, xanthines, retinoids, plant extracts of the beta-sitosterin type, thymus extracts.

Leaving aside the drugs for systemic administration, characterized by their specificity of action and efficacy-tolerability ratio, whose use involves different technical problems and that are not of interest to this invention, only few of the other products used so far have showed some activity, and it is however showed only in particular types of alopecia and in some phases of the phenomenon.

Also considering the therapies now defined as "future prospects", such as the possible use of the Hair Growth Factor HrGF, unsuitable in any case for topical use, the conclusion is that none of the therapies currently in use or under development is based on topical administration of active principles capable of developing a synergistic corrective effect on the various factors that contribute to the genesis and progression of several forms of alopecia: hormonal, dysmetabolic, immunological, phlogogenic, of reduced cellular function, and seborrheic and so on.

EP 1 344 529 discloses a composition for treating male pattern baldness, which includes an ingredient, e.g. an herbal extract, inhibiting aromatase activity, i.e. reduces the rate at which testosterone is converted to oestradiol and, at the same time, increases the level of testosterone.

WO 2008/009960 discloses the use of anti-testosterone antibodies for various therapeutic uses.

It is therefore still felt the need of such a composition, that is effective in the topical treatment of alopecia.

### Summary of the invention

The Applicant has now found a new pharmaceutical composition for topical use, which is particularly effective in inhibiting the hair loss and in facilitating the hair regrowth after topical administration in subjects suffering of alopecia of different aetiology, thanks to the synergic effects of immunomodulating cellular fragments and of anti-testosterone antibodies, both comprised in the composition.

Thanks to this composition the different contributing factors involved in hair loss and in their lack of regrowth, are removed, and a correct prophylaxis is carried out, together with a treatment of most diseases related to hair loss and lack of hair regrowth.

Subject of the present invention is therefore a pharmaceutical composition for topical use, comprising as active principles at least a non-specific immunomodulator of microbial origin obtained from *Corynebacterium granulosum* by means of a procedure including delipidation, crushing and purification of the cellular wall and anti-testosterone polyclonal antibodies.

Further subjects of the invention are the process for preparing this composition and the kit of compositions of immunomodulators and antibodies for the preparation of the above said composition.

Further characteristics and advantages of the invention will be illustrated in the following detailed description.

### Detailed description of the invention

The pharmaceutical composition of the invention comprises non-specific immunomodulators of microbial origin, preferably obtained from *Corynebacterium granulosum* by means of a procedure including delipidation, crushing and purification of the cells of *C*. *granulosum* yielding to fragments of cellular wall having immunomodulating action towards subcutaneous-submucosal macrophage-like immunocompetent cells, in particular Langerhans cells and keratinocytes.

Particularly preferred for the use in the present invention is the insoluble fraction so called P40, consisting of glycoproteins and peptidoglycans of the cellular wall of *C*. *granulosum* obtained from the microorganism by the delipidation, crushing and purification procedure disclosed by B. Bizzini et al. in Med. Mal. Inf., 1978, 8, 408.

The ability of certain microorganisms or parietal fractions thereof to non-specifically stimulate the immune system is already described in literature. In particular the P40 fraction obtained from *Corynebacterium granulosum* has been studied, in experiments and clinical trials, but only for parenteral administration. When administered by subcutaneous or intramuscular route, P40 was able to develop a wide range of pharmacological effects such as the inhibition of tumor growth in mice [E.H. Relyverd et al., Compt. Rend. Acad. Sci., Paris, 1980, 273, 39], adjuvant effects [E. Henocq et al., Med. et Mal. Unfect, 1978, 8, 415], "*in vivo"* antimicrobial effects [D. Mathieu et al., Biomédicine, 1982, 36, 420], antiviral effects [M. Fattal-German et al., Biomed. Pharmacother., 1992, 77, 115]. Furthermore, always by parenteral administration, P40 has also been widely tested in clinical trials for the treatment of various types of infections and breast cancer.

Now the Applicant has surprisingly found that the P 40 fraction exhibits some of its properties even by topical administration maintaining, when administered by this route, its ability to stimulate the reticulo-endothelial system, to activate the complement, to promote the endogenous biosynthesis of interferons, and to positively interact with the cytokines cascade. In experimental studies a significant immunostimulating activity results from the topical administration of this product.

The immunomodulator may be formulated for instance with hydroxypropylcellulose thus obtaining a composition of appropriate viscosity for topical administration.

In combination with the immunomodulator, the composition of the invention comprises polyclonal anti-testosterone antibodies that, following topical administration on the scalp, showed to be able to complex the metabolically active androgenic hormones (testosterone, dihydrosterone and androstandiol), that accumulate the excess amount in the hair follicles and sebaceous glands. Immunocomplexes are formed that inactivate hormones and are in their turn eliminated: those present in the sub-cellular layers, by phagocytosis carried out by macrophage-like submucosal cells, suitably activated by immunostimulating cellular fragments; while those present in the surface layers, just by action of washing.

Polyclonal anti-testosterone antibodies suitable for use in the present compositions may be obtained for instance from sheep, goats or cows, and employed in a purified form starting from the animal's serum and after stabilization, for example by chemical conjugation with dextran, or they may be used as components of the globulins present in milk. In this second instance, a further advantage is represented by the fact that some protein components of milk may act as substrate for the development of energetic mechanisms involved in the cellular functions and hair regrowth.

According to a particularly preferred embodiment of the invention the anti-testosterone polyclonal antibodies are formulated by adding hyaluronic acid and derivatives thereof, salts and esters, such as sodium hyaluronate; this facilitates the transport of antibodies at subcellular level, assisting in the complete elimination of antigens.

The content of hydroxypropylcellulose and of hyaluronic acid in the present composition is determined so as to allow the best possible contact between the active principles and the skin tissue. Further excipients and adjuvants, that are pharmaceutically acceptable and commonly used in the topical pharmaceutical compositions, such as emollients, thickening agents, ionic or non ionic surfactants, emulsifying agents, bacteriostatic agents, dyes, perfumes, and combinations thereof, may be added to the present composition.

The present composition is formulated with a formulation suitable for topical use. According to a particular embodiment of the invention the two active principles, the immunostimulating and the antibody principles - in particular when this second component is present in the form of milk globulins - are kept separated until use for instance in a bottle with pierceable plug where for instance the immunostimulating component is contained in the bottle and the antibody component is contained in the pierceable plug, the two compositions being homogenized before application. It is subject of the present invention a kit of compositions comprising a topical formulation of a non specific immunomodulator of microbial origin; and a powder formulation of polyclonal anti-testosterone antibodies, to be mixed together at time of use to yield the composition for topical administration according to the invention.

The dosage of immunomodulating cellular fragments and of anti-testosterone antibodies may varies according to the severity of the pathology to be treated, of the circulating androgen hormone levels, and it takes into account the possibility to carry out an attack therapy or a maintenance therapy. The attack therapy averagely lasts ten/fifteen days; the maintenance therapy is on a quarterly basis and has an average duration of five/seven days.

By way of example the attack formulation containing purified antibodies may comprise from 1.5 to 4.0 ug of anti-testosterone antibodies conjugated to dextran and from 5.0 to 15.0 mg of hyaluronic acid; and from 50.0 to 200.0 ug of cellular fragments of *C*. *granulosum*; while the same formulation with milk globulins, comprises for instance an amount of milk globulins ranging from 10.0 to 50.0 mg, the further components being as said above. In the maintenance formulations the composition comprises for instance from 10.0 to 50.0 mg of milk globulins and from 20.0 to 100.0 ug of cellular fragments of *C*. *granulosum,* with the above said amounts of hyaluronic acid.

Thanks to the synergic use of the immunostimulating cellular fraction P40 and of the polyclonal anti-testosterone antibodies, the present composition has given significant results in the prevention of baldness correlated to different forms of alopecia, as well as in assisting the hair regrowth in bald subjects. The present composition has also proven its efficacy in treating dysmetabolic-pathogenetic side forms, such as seborrhoea, seborrheic dermatitis and dandruff.

Contrary to traditional forms of treatment in use to date or to those under development described above, the present composition is able to act synergistically on the various problems involved in the different forms of alopecia, thus obtaining a result of overall treatment. In particular, the present composition has proved to have the following effects:
- neutralization of testosterone and of the active metabolites thereof, dihydrotestosterone and androstandiol, present in excess in hair follicles and sebaceous glands; reactivation of the functions of the keratinocytes with promotion, among other things, of the biosynthesis of endogenous HrGF;
- elimination of the phlogogenic processes with reactivation of cellular functions and consequent rebalancing of the glucose metabolism *in situ*;
- promotion of the immune response, in particular of the cell-mediated response; elimination of sebum in excess, of sebum deposits and of its consequent hardening;
- regulation of the microbial balance of the scalp;
- reduction, as a consequence, of the hair loss process and favoring of hair regrowth, very significantly, by topical administration "in situ" of an association between a non-specific immunomodulator of bacterial origin and polyclonal anti-testosterone antibodies.

The following examples are provided by way of illustration.

### Example 1 - Preparation and characterization of active principles

### 1.1. Immunomodulator of bacterial origin P40

### Preparation of cellular fragments of Corynebacterium granulosum having non-specific immunomodulating action (P40):

The cellular fragments having immunomodulating action are prepared, starting from the culture of *Corynebacterium granulosum,* according to the method described by B. Bizzini [Med. Mal. Inf., 1978, 8, 408] and comprising the following steps:
- growth of the microorganism under anaerobiosis at 37°C for 48 - 60 hours, with a yield of dry mass of approx. 3%;
- collection of the microorganisms by centrifugation and washing;
- delipidation in Soxhlet against methanol - ether, chloroform, methanol - chloroform: yield 2% of the total;
- crushing by sonication - 3 cycles for 1 min. each;
- collecting by centrifugation and washing;
- separation of the active fraction by velocity gradient centrifugation, with a yield of approx. 20% of the delipidated product.

### Determination of the biological properties:

The following experiments have been carried out:
1. Splenomegaly induced in mice:
   7 days after a single subcutaneous administration of 500 ug of P40 obtained as described above, a Swiss mouse was sacrificed and the weight increase of its spleen was measured, as an indication of stimulation of the reticulo-endothelial system. The increase of weight of the spleen, with respect to control animals, was higher than 40%.
2. Granulopexic activity - K/Ko index:

The granulopexic activity was determined in accordance with the method described by Halpern et al. in Ann. Inst. Pasteur, 1951, 80, 582, by a single intravenous administration in Swiss mice of 250 ug / mouse of P 40 obtained as described above and, on the 6th day from the treatment, the metabolic fate of the china ink particles injected was controlled as index of stimulation of the reticulo-endothelial system. The value of the K/Ko index was higher than 4.5.

### Analytical characterization:

The following analytical characterizations were carried out:

### 1. Concentration of muramic acid

The concentration of muramic acid was determined by the Spackman method, as described in Anal. Chem., 1958, 30, 1190, resulting comprised between 8.0 and 12.0 g / 100 g of dry weight.

### 2. Concentration of diaminopimelic acid (DAP)

The concentration of diaminopimelic acid (DAP) was determined by automatic aminoacidic analysis by subtraction of methionine from the rate DAP + methionine, resulting comprised between 2.5 and 3.5 g / 100 g of dry weight.

### 3. Hexosamines

Hexosamines were measured by the Elson method, described in Biochem. L., 1933, 27, 1824, resulting comprised between 8.0 and 11.5 g / 100 g of dry weight.

### 4. Neutral sugars

Neutral sugars were measured by the anthrone method as disclosed by P. Albesheim et al. in Carbohydrate Res., 1967, 5, 340, resulting comprised between 12.0 and 15.0 g /100 g of dry weight.

### Pharmacological characteristics:

The following experiments have been carried out:

### 1. Experimental infection by Listeria monocitogenes in mice:

The experimental infection is induced in mice by intravenous administration of 1.0 x 10⁵ of bacteria of a 18 hours culture. The treatment with activated P40 was obtained as described above, by subcutaneous administration of 250 ug *pro die* for three days before the experimental infection. The percentage of protection from death due to infection, in a period of time of ten days, was approximately 75%.

### 2. UV-induced erythema in the rat (cutaneous-mucosal antiphlogogenic effect)

The shaved backs of rats were exposed to UV rays until a large erythematosus state appeared, then for three days a gel containing P40 was administered on the injured area by subsequent applications of 50 ug of P40 / application. In the animals so treated the erythematosus state was resolved in significantly shorter times than in control animals.

### 3. Adjuvant ability in mice

The increase in the production of hemagglutinins was evaluated in response to the administration of sheep erythrocytes in mice according to the method described in Raynaud et al. in Ann. Inst. Pasteur, 1972, 122, 695. Sheep red blood cells were administered at the dosage of 5 x 10⁶ cells. The intraperitoneal administration of 500 ug/mouse of P40 induced an increase in the production of antibodies of five orders of magnitude higher.

### 4. Delayed hypersensitivity reaction in Guinea pigs

In Guinea pigs an antigen (ovalbumin) in Freund's incomplete adjuvant added with 200 ug/animal of the active principle, was subcutaneously administered. The development of a typical skin reaction of delayed hypersensitivity, was monitored at the site of intradermal injection of the antigen, compared to control animals that have just received the antigen in Freund's incomplete adjuvant. This reaction was observed after 20 days from sensitization only in animals treated with P40.

### 5. Influence on the speed of hair growth in rabbits

Rabbits are deeply shaved in some back areas, and thereafter in the shaved area P40 is applied in the form of a gel containing 50 ug / application for three consecutive days. The speed of hair regrowth is then monitored. In the treated animals the hair regrowth time up to normal conditions, is reduced by 50%.

### 1.2. Anti-testosterone antibodies

### Preparation of immunogens

### Work immunogen: ovoalbumin-testosterone

The immunogens are prepared according to traditional synthetic methods by reaction of an anhydride group of the haptene with an amine group in the carrier protein (ovoalbumin) as described for example in B.F. Erlanger et al., J. Biol. Chem., 1957,228,713.

ELISA test for evaluating the "responder" status and the antibodies properties The kit is prepared according to traditional methods, as described for instance in D.M. Weir, Handbook of Exp. Immunol., 1978, Blackwell Sci. Pubi., Oxford.

The serum of the animal is contacted with the control immunogen (human albumin -testosterone) adhered to the well of a microplate.

The presence of anti-testosterone antibodies is revealed by adding an enzyme-conjugated antibody and by subsequently evaluating the colour development in the presence of a suitable substrate.

### Average D.O. at 405 nm of a solution 1 : 10 of the serum = 1,500

### Immunization protocol of animals and antisera production

The animal, a sheep, is treated according to the traditional immunization procedures described for instance in A. Johnstone et al., Immunochem. in Practice, 1982, Blackwell Sci. Pubi., Oxford, by subcutaneous administration in the sensitization phase of the work immunogen (5 mg/kg) associated to Freund's complete adjuvant and, in the booster phase the work immunogen (2 mg/kg) associated to Freund's incomplete adjuvant.

Every thirty days a subsequent booster and a control blood sampling is carried out, until the "responder" (i.e. the animal producing antibodies) state is reached.

### Purification of anti-testosterone antibodies (IgG) from the blood and their stabilization by chemical covalent conjugation to polymers of the dextran family

This test was carried out according to traditional methods known in the art as described for instance in A. Johnstone et al., Immunochem. in Practice, 1982, Blackwell Sci. Pubi., Oxford. Immunoglobulins are precipitated from serum by adding ammonium sulphate in excess. After centrifugation and washing they are re-dissolved in water and purified by chromatography on DEAE-cellulose, then concentrated again.

Their conjugation, aimed at stabilizing them, is carried out by reaction with dextran pre-activated with cyanogen bromide (BrCN), as described in L. Kagedal et al., Acta Chem. Scandinav., 1871,25, 1855.

### Preparation of the milk extract from vaccinated animals containing the anti-testosterone antibodies

For this preparation the method for purifying the egg immunoglobulins provided by Jensenius and described in J. Jensenius et al., J. Immunol. Metri., 1981, 46, 63, was followed after suitable modifications. This method comprises the delipidation of the starting product, the proteins isolation and the subsequent separation of immunoglobulins.

The purified product is then dried by spray drying and stabilized with preservatives.

### Example 2 - Description of the formulations

The formulation used for the present compositions is that of a bottle with a pierceable plug, given the technological features of the active principles.

### Example 2A - Topical pharmaceutical form for attack treatment containing purified anti-testosterone antibodies, conjugated to dextran

Bottle and pierceable plug each containing:
a. pierceable plug:

| | |
|---|---|
| Anti-testosterone antibodies - dextran | ug 1.8 |
| hyaluronic acid | mg 9.0 |
| sodium chloride | enough for filling the plug |

b. bottle:

| | |
|---|---|
| *C*. *granulosum* cellular fragments | ug 100.0 |
| hydroxypropylcellulose | mg 500.0 |
| distilled water | enough until ml 10.0 |

### Example 2B - Topical pharmaceutical attack form containing a mixture of milk globulins of the animal vaccinated against testosterone

Bottle with pierceable plug containing:
a. pierceable plug:

| | |
|---|---|
| globulins from sheep milk | mg 20.0 |
| hyaluronic acid | mg 9.0 |
| sodium chloride | enough for filling the plug |

b. bottle:

| | |
|---|---|
| *C*. *granulosum* cellular fragments | ug 100.0 |
| hydroxypropylcellulose | mg 500.0 |
| distilled water | enough until ml 10.0 |

### Example 2C - Topical pharmaceutical form for maintenance

Bottle with pierceable plug containing:
a. pierceable plug:

| | |
|---|---|
| globulins from sheep milk | mg 20.0 |
| hyaluronic acid | mg 9.0 |
| sodium chloride | enough for filling the plug |

b. bottle:

| | |
|---|---|
| *C. granulosum* cellular fragments | ug 50.0 |
| hydroxypropylcellulose | mg 500.0 |
| distilled water | enough until ml 10.0 |

The modes of use of the present compositions can be summarized as follows:
break the plug mixing all components, apply on the skin and leave in contact for at least one hour before washing.

### Example 3 - Analytical control of the compositions

The compositions in the pierceable plug were subjected to ELISA tests for the evaluation of the content of anti-testosterone antibodies; and to glucosamine analysis by ionic exchange chromatography with borate buffer, as described in Y.C. Lee et al., J. Biol. Chem., 1972, 247, 5753.

The compositions in the bottle were examined for measuring the amount of muramic acid in the suspension.

### Example 4 - Pharmacological-clinical experimental part

### 4.1. Efficacy control towards the hair defluvium in subjects suffering of hypotrichia in evolutionary stage

Twenty volunteers, ten male and ten female, were selected; they were all suffering of hair loss in small amount, due to adverse conditions such as stress, surgical interventions, fever conditions, delivery and nursing.

After an objective dermatological examination according to what described for instance in M.K. Hordinsky, Dermatol. Clin., 1987, 3, 483, with the aim of defining the amount of hair lost, their structural features and the general state of the subjects, the patients were subject to a treatment with the present compositions. The treatment was activated with the pharmaceutical attack form B) by an application *pro die* for ten days. Already from the sixth day all patients showed a significant reduction of hair defluvium, which was absent at all at the end of treatment. The anatomopathological tests performed on the hair, always at the end of treatment, showed a return to the normal conditions for the physiological and functional features of the hair.

### 4.2. Efficacy control towards hair loss and hair regrowth in subjects having alopecia of different aetiology in advanced phase

Twenty male volunteers were selected, all having an ascertained diagnosis of androgenic alopecia or of alopecia aerata.

The nature and severity of pathogenesis has been determined on the basis of a dermatological objective examination, according to what described for instance in M.K. Hordinsky, Dermatol. Clin., 1987, 3, 484.

Patients were subjected to a topical treatment for 15 days with the form A) of the composition.

Subsequently every three months, the same patients were treated with the maintenance form C) of the composition, administered every 7 days, for a total of 12 months.

A periodic control was carried out of the extent of hair loss, of the anatomical-morphological features of the hair, of the presence and dimensions of bald spots, of the speed and size of the regrowth.

Already starting from the end of the attack treatment, in the 100% of the patients a great reduction of the hair loss and of the bald spots was observed, and in 70% of the same patients the hair regrowth process was evident.

At the end of the 12-months treatment the scalp has returned to a normal condition in 70% of the patients, while the remaining subjects showed a significant amelioration.

The anatomical-morphological features of the re-grown hair and scalp were also normal.

### 4.3. Efficacy control towards seborrheic dermatitis

Twenty male volunteers were selected, all having an ascertained diagnosis of seborrheic dermatitis.

The presence, extent and severity of the pathology was determined on the basis of objective parameters (presence of yellowish and greasy scales associated with erythema of the scalp, presence of crusted formations and chemical analysis of the qualitative composition of sebum) and on the basis of subjective parameters (itching).

The subjects were subjected to a daily administration, for 10 consecutive days, of the composition of the invention in the pharmaceutical form B) provided for the attack treatment. Already on the fifth day of treatment the itching and erythema of the scalp disappeared in all patients.

At the end of treatment, always for all patients treated, there were neither scales nor crusted formations. Furthermore, the chemical analysis carried out on samples taken with a tampon showed the return within normal values of the qualitative composition of sebum.

## Claims

1. A pharmaceutical composition for topical use, comprising as active principles at least a non-specific immunomodulator having microbial origin and polyclonal anti-testosterone antibodies, **characterised in that** said non-specific immunomodulator consists of insoluble fragments of the cellular wall of *Corynebacterium granulosum* delipidated, crushed and purified.

2. The composition according to claim 1, wherein said polyclonal anti-testosterone antibodies are produced in sheep, and used after purification of immunoglobulins from serum of the "responder" animal.

3. The composition according to claim 1, wherein said polyclonal antibodies antitestosterone are produced in sheep, and used as component of the globulins in the milk of the "responder" animal.

4. The composition according to any of the preceding claims, further comprising one or more pharmaceutically acceptable adjuvants and/or excipients, such as emollients, thickeners, ionic or non ionic surfactants, emulsifiers, bacteriostatic agents, colouring agents, perfumes, and combinations thereof.

5. The composition according to claim 4, wherein said adjuvants and/or excipients are selected from among hyaluronic acid and derivatives thereof, sodium hydroxypropylcellulose and combinations thereof.

6. The composition according to the preceding claims, comprising from 1.5 to 4.0 ug of anti-testosterone antibodies conjugated to dextrane, from 50.0 to 200.0 ug of cellular fragments of *C*. *granulosum,* and from 5.0 to 15.0 mg of hyaluronic acid.

7. The composition according to the preceding claims, comprising from 10.0 to 50.0 mg of said globulins from milk, from 50.0 to 200.0 ug of cellular fragments of C. *granulosum,* and from 5.0 to 15.0 mg of hyaluronic acid.

8. The composition described in claims from 1 to 7 for use as a medicament in the prevention and treatment of hypotrichia and alopecia and in the treatment of other disorders involving the scalp, such as seborrheic dermatitis and dandruff.

9. A process for the preparation of the composition described in claims 1 -7 comprising the following steps:
a) formulation for topical use of the non-specific immunomodulator of microbial origin according to claim 1;
b) formulation of polyclonal antibodies anti-testosterone;
c) homogenization of the two formulations obtained from steps a) and b) at the time of use.

10. The process according to claim 9, wherein said formulation in step a) is obtained by mixing said immunomodulator with hydroxypropylcellulose, and optionally adding distilled water to the desired final volume.

11. The process according to claim 9, wherein said formulation in step b) is obtained by mixing said polyclonal antibodies with hyaluronic acid or derivatives thereof, and optionally adding sodium chloride to the desired final volume, and maintaining the so obtained powder formulation separated from the formulation in step a) until the time of use.

12. A kit of compositions for the preparation of the composition described in claims from 1 to 7 comprising a topical formulation of the non-specific immunomodulator of microbial origin according to claim 1; and a powder formulation of polyclonal anti-testosterone antibodies, to be mixed at the time of use so as to obtain a composition for topical use.

## Patentansprüche

1. Pharmazeutische Zusammensetzung zur topischen Verwendung, enthaltend als aktives Prinzip wenigstens einen unspezifischen Immunmodulator, der einen mikrobiellen Ursprung und polyklonale Anti-Testosteron Antikörper hat, **dadurch gekennzeichnet, dass** der unspezifische Immunmodulator aus unlösbaren Fragmenten der Zellwand von *Corynebacterium granulosum,* zerfallen, zerkleinert und gereinigt, besteht.

2. Zusammensetzung nach Anspruch 1, wobei die polyklonalen Anti-Testosteron Antikörper in Schafen produziert werden und nach der Reinigung von Immunglobulin von Serum des "Responder"-Tiers verwendet werden.

3. Zusammensetzung nach Anspruch 1, wobei das polyklonale Antikörper-Anti-Testosteron in Schafen produziert werden und als Komponente des Globulins in der Milch des "Responder"-Tiers verwendet werden.

4. Zusammensetzung nach einem der vorhergehenden Ansprüche, ferner enthaltend einen oder mehrere pharmazeutisch akzeptable Hilfsmittel und/oder Bindemittel, wie Weichmacher, Verdicker, ionische oder nichtionische Tenside, Emulgatoren, bakteriostatische Mittel, Färbungsmittel, Duftmittel und Kombinationen davon.

5. Zusammensetzung nach Anspruch 4, wobei die Hilfsmittel und/oder Bindemittel ausgewählt sind unter Hyaluronsäure und Derivaten davon, Natrium-Hydroxypropylzellulose und Kombinationen davon.

6. Zusammensetzung nach einem der vorhergehenden Ansprüche, enthaltend von 1,5 bis 4,0 ug von Anti-Testosteron Antikörpern konjugiert mit Dextran, von 50,0 bis 200,0 ug von zellularen Fragmenten von *C*. *granulosum,* und von 5,0 bis 15,0 mg Hyaluronsäure.

7. Zusammensetzung nach einem der vorhergehenden Ansprüche, enthaltend von 10,0 bis 50,0 mg der Globulins von Milch, von 50,0 bis 200,0 ug von zellularen Fragmenten von *C*. *granulosum,* und von 5,0 bis 15,0 mg Hyaluronsäure.

8. Zusammensetzung, wie in den Ansprüchen 1 bis 7 angegeben, zur Verwendung als Medikament bei der Prävention und Behandlung von Hypotrichosis und Alopezie und bei der Behandlung von anderen Störungen betreffend die Kopfhaut, wie die seborrhöische Dermatitis und Schuppen.

9. Verfahren zur Vorbereitung der Zusammensetzung, die in den Ansprüchen 1 bis 7 beschrieben ist, enthaltend die folgenden Schritte:
a) Zubereitung zur topischen Verwendung des unspezifischen Immunmodulators von mikrobiellem Ursprung nach Anspruch 1;
b) Zubereitung von polyklonalem Antikörper-Anti-Testosteron;
c) Homogenisierung der zwei Zubereitungen, die von den Schritten a) und b) erhalten wurden, zum Zeitpunkt der Verwendung.

10. Verfahren nach Anspruch 9, wobei die Zubereitung im Schritt
a) durch Mischen des Immunmodulators mit Hydroxypropylzellulose und optionalem Hinzufügen von destilliertem Wasser bis zum gewünschten endgültigen Volumen erhalten wird.

11. Verfahren nach Anspruch 9, wobei die Zubereitung im Schritt b) durch Mischen der polyklonalen Antikörper mit Hyaluronsäure oder Derivaten davon und optionalem Hinzufügen von Natriumchlorid bis zum gewünschten endgültigen Volumen und Getrennthalten der so erhaltenen Pulverzubereitung von der Zubereitung im Schritt a) bis zum Zeitpunkt der Verwendung erhalten wird.

12. Kit von Zusammensetzungen zur Vorbereitung der Zusammensetzung, die in den Ansprüchen 1 bis 7 beschrieben ist, enthaltend die topische Zubereitung des unspezifischen Immunmodulators von mikrobiellem Ursprung nach Anspruch 1;
und eine Pulverzubereitung von polyklonalen Anti-Testosteron Antikörpern, um zum Zeitpunkt der Verwendung gemischt zu werden, um eine Zusammensetzung zur topischen Verwendung zu erhalten.

## Revendications

1. Une composition pharmaceutique pour usage local, comportant comme principes actifs au moins un immunomodulateur non spécifique ayant une origine microbienne et des anticorps polyclonaux anti-testostérone, **caractérisée en ce que** ledit immunomodulateur non spécifique se compose de fragments insolubles de la paroi cellulaire de *Corynebactérie granulosum* délipidée, broyée et épurée.

2. La composition selon la revendication 1, où lesdits anticorps polyclonaux anti-testostérone sont produits chez les moutons, et utilisés après purification d'immunoglobulines extraites du sérum de l'animal "répondeur".

3. La composition selon la revendication 1, où lesdits anticorps polygonaux anti-testostérone sont produits chez les moutons, et utilisés comme composants des globulines dans le lait de l'animal "répondeur".

4. La composition selon l'une quelconque des revendications précédentes, comportant en outre un ou plusieurs adjuvants et/ou excipients pharmaceutiquement acceptables, comme des émollients, des épaississants, des agents tensio-actifs ioniques ou non ioniques, des émulsifiants, des agents bactériostatiques, des agents de coloration, des parfums, et des combinaisons de ceux-ci.

5. La composition selon la revendication 4, où lesdits adjuvants et/ou excipients sont choisis parmi l'acide hyaluronique et ses dérivés, l'hydroxypropylcellulose de sodium et des combinaisons de ceux-ci.

6. La composition selon les revendications précédentes, comprenant de 1,5 à 4,0 ug d'anticorps antitestostérone conjugués à du dextrane, de 50,0 à 200,0 ug de fragments cellulaires de *C*. *granulosum,* et de 5,0 à 15,0 mg d'acide hyaluronique.

7. La composition selon les revendications précédentes, comprenant de 10,0 à 50,0 mg de dites globulines de lait, de 50,0 à 200,0 ug de fragments cellulaires de *C*. *granulosum,* et de 5,0 à 15,0 mg d'acide hyaluronique.

8. La composition décrite dans les revendications de 1 à 7 pour usage comme médicament dans la prévention et le traitement de l'hypotrichose et de l'alopécie et dans le traitement d'autres désordres impliquant le cuir chevelu, tel que la dermatite séborrhéique et les pellicules.

9. Un procédé pour la préparation de la composition décrite dans les revendications 1-7, comportant les étapes suivantes :
a) élaboration pour usage local de l'immunomodulateur non spécifique d'origine microbienne selon la revendication 1 ;
b) élaboration d'anticorps polyclonaux anti-testostérone ;
c) homogénéisation, au moment de l'utilisation, des deux préparation obtenues lors des étapes a) et b).

10. Le procédé selon la revendication 9, où ladite élaboration lors de l'étape a) est obtenue en mélangeant du dit immunomodulateur avec de l'hydroxypropylcellulose, et optionnellement en ajoutant de l'eau distillée jusqu'au volume final désiré.

11. Le procédé selon la revendication 9, où ladite élaboration lors de l'étape b) est obtenue en mélangeant lesdits anticorps polyclonaux avec de l'acide hyaluronique ou ses dérivés, et optionnellement en ajoutant du chlorure de sodium jusqu'au volume final désiré, et en maintenant le produit ainsi obtenu sous forme de poudre séparé de celui élaboré dans l'étape a) jusqu'au moment de l'utilisation.

12. Un kit de compositions pour la préparation de la composition décrite dans les revendications 1 à 7, comportant une préparation à usage local de l'immunomodulateur non spécifique d'origine microbienne selon la revendication 1 ; et une préparation en poudre d'anticorps polyclonaux anti-testostérone, pour être mélangées au moment de l'utilisation afin d'obtenir une composition à usage local.
